(19) Europäisches Patentamt
European Patent Office
Office européen des brevets

(11) Veröffentlichungsnummer: **0 047 451**
**B1**

# (12) EUROPÄISCHE PATENTSCHRIFT

(45) Veröffentlichungstag der Patentschrift: **01.02.84**

(51) Int. Cl.³: **C 07 C 145/02,** C 07 D 295/18, A 01 N 47/04

(21) Anmeldenummer: **81106725.5**

(22) Anmeldetag: **28.08.81**

## (54) Fluor- und Chlor-methyl-thioamino-benzamide, ein Verfahren zu ihrer Herstellung und ihre Verwendung.

(30) Priorität: **09.09.80 DE 3033863**

(43) Veröffentlichungstag der Anmeldung: **17.03.82 Patentblatt 82/11**

(45) Bekanntmachung des Hinweises auf die Patenterteilung: **01.02.84 Patentblatt 84/5**

(84) Benannte Vertragsstaaten: **BE CH DE FR GB IT LI SE**

(56) Entgegenhaltungen: **DE - B - 1 543 614**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Ritter, Helmut, Dr., Bodelschwinghstrasse 16, D-4150 Krefeld 1 (DE)**
Erfinder: **Paulus, Wilfried, Dr., Deswatinesstrasse 90, D-4150 Krefeld 1 (DE)**
Erfinder: **Kühle, Engelbert, Dr., von Bodelschwing-Strasse 42, D-5060 Bergisch-Gladbach 2 (DE)**
Erfinder: **Genth, Hermann, Dr., Am Heckerhof 60, D-4150 Krefeld 1 (DE)**

**EP 0 047 451 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Fluor- und Chlor-methyl-thioamino-benzamide, ein Verfahren zu ihrer Herstellung und ihre Verwendung

Die Erfindung betrifft Fluor- und Chlor-methyl-thioamino-benzamide, ein Verfahren zu ihrer Herstellung und ihre Verwendung in mikrobiziden Mitteln.

Aus der DE-AS 1 543 614 sind N-(Trihalogenmethylthio)-N-trifluormethyl-aminobenzamide und ihre fungizide Wirkung bekannt. Für die technische Anwendung ist ihre Wirkung nicht immer befriedigend.

Die neuen Fluor- und Chlor-methyl-thioamino-benzamide sind gekennzeichnet durch die Formel

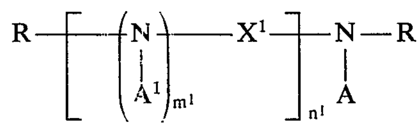

(I)

worin

$X^1$ für gegebenenfalls substituiertes Alkylen, Arylen, Alkarylen, wobei die Alkylen- und/oder Arylen- und/oder Alkarylengruppen über Oxo-, Thio-, Carboxo-, Carbonamido- und Sulfoxogruppen verknüpft sein können,
steht,

$A^1$ den Rest

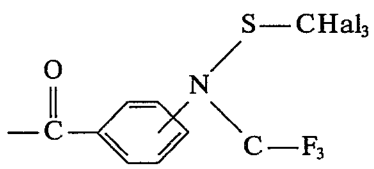

worin
Hal für Halogen steht,
bedeutet,

R in den unterschiedlichen Substitutionen gleich oder verschieden ist und für Wasserstoff, Alkyl oder Aryl steht oder für den Fall, daß $X^1$ Ethylen bedeutet und $m^1$ und $n^1$ für die Zahl 1 stehen, durch Verknüpfung mit einem zweiten R eine Ethylenbrücke bildet,

$m^1$ eine Zahl von 1 bis 5 bedeutet und die Anzahl der Verknüpfungen von

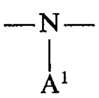

mit $X^1$ angibt und

$n^1$ eine Zahl von 1 bis 100 bedeutet.

Alkylen kann ein geradkettiger, cyclischer oder verzweigter zweibindiger Kohlenwasserstoffrest mit 2 bis 13 Kohlenstoffatomen sein. Als Alkylenreste seien beispielsweise Ethylen, Propylen, Isopropylen, Butylen, Isobutylen, Pentylen, Isopentylen, Hexylen, Cyclohexylen, Dicyclohexylenmethan und Isohexylen genannt. Bevorzugte Alkylenreste sind der Ethylen- und der Hexylen-Rest.

Arylen kann aus 1 bis 5 Phenylenkernen bestehen, die entweder linear verknüpft oder kondensiert sind. Beispielsweise seien die folgenden Arylene genannt: Phenylen, Diphenylen, Triphenylen, Anthrachinonen und Naphthylen.

Bevorzugte Arylene sind Phenylen, Biphenylen und Naphthylen.

Alkarylen ist im allgemeinen ein durch Verknüpfung von einem oder mehreren, bevorzugt einem bis fünf, Alkylenen mit Arylenen gebildeter Rest, bei dem Alkylen aus 1 bis 6 Kohlenstoffatomen und Arylen aus 1 bis 5 linear verknüpften oder kondensierten Phenylkernen besteht. Beispielsweise seien die folgenden Alkarylene genannt: Methylphenylen, Isopropylphenylen, 2,2-Bis-phenylenpropan, 1,1'-Bisphenylencyclohexan, Diphenylenmethan, 3,3'-Dichlor-4,4'-diphenylenmethan, Triphenylenmethan, Xylylen.

Bevorzugte Alkarylene sind Methylphenylen, Diphenylenmethan, 2,2-Bis-phenylenpropan.

Die obengenannten Alkylen- und/oder Arylen- und/oder Alkarylengruppen können auch über Oxo-, Thio-, Carboxo-, Carbonamido- und Sulfoxogruppen verknüpft sein. Beispielsweise seien hier die folgenden Reste genannt: Diphenylenether, Diphenylenketon, Diphenylensulfid, Diphenylensulfoxid, Diphenylensulfon, N-Phenylenbenzoylamid.

Bevorzugte Reste sind Diphenylenether, Diphenylensulfid.

Halogen in den Halogenmethylgruppen des Restes $A^1$ ist im allgemeinen Fluor und/oder Chlor. Beispielsweise seien Trifluormethyl, Trichlormethyl, Difluor-chlor-methyl und Dichlor-fluor-methyl genannt.

Alkyl ist im allgemeinen ein geradkettiger oder verzweigter Kohlenwasserstoffrest mit 1 bis 6 (Niederalkyl) Kohlenstoffatomen. Beispielsweise seien Methyl, Ethyl, Propyl, Isopropyl, Butyl, Isobutyl, Pentyl, Isopentyl, Hexyl und Isohexyl genannt. Bevorzugte Alkylreste sind Methyl und Ethyl.

Aryl besteht im allgemeinen aus einem oder mehreren, bevorzugt bis zu 5, linear verknüpften oder kondensierten Phenylresten. Beispielsweise seien für Aryl die folgenden Reste genannt: Phenyl, Chlorphenyl, Nitrophenyl, Diphenyl, Diphenylmethan, Naphthyl.

Bevorzugtes Aryl ist Phenyl.

In dem Fall, daß $X^1$ Ethylen bedeutet und $m^1$ und $n^1$ für die Zahl 1 stehen, ist es auch möglich, daß zwei Reste R zu einer Ethylenbrücke verknüpft sind und daß so ein Piperazinring gebildet wird.

Die Reste $X^1$, $A^1$ und R können außerdem durch übliche Reste substituiert sein. Beispielsweise seien hier die folgenden Reste genannt: Nitro, Halogen wie Fluor, Chlor, Brom und Jod, Carbonsäureamid, N-Alkylcarbonsäureamid mit 1—6 C-Atomen, N-Phenylcarbonsäureamid, Carbonsäureester mit 1—6 C-Atomen, Carbonsäure, Sulfonsäure, Fluoralkyl mit 1—6 C-Atomen, Cyano und Methoxy.

Als bevorzugte Fluor- und Chlor-methyl-thioamino-benzamide seien Verbindungen der Formel

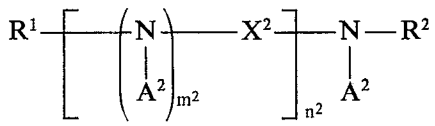

(II)

worin

$X^2$ für gegebenenfalls substituiertes geradkettiges oder verzweigtes Alkylen mit 2 bis 6 Kohlenstoffatomen, Arylen mit 1 bis 5 linear verknüpften oder kondensierten Phenylenkernen, Alkarylen, wobei Arylenkerne des Alkarylens durch geradkettiges oder verzweigtes Alkylen mit 1 bis 6 Kohlenstoffatomen verbunden sein können, und wobei die Alkylen- und/oder Arylen- und/oder Alkarylengruppen über Oxo-, Thio-, Carboxo-, Carbonamido- und Sulfoxogruppen verknüpft sein können, steht,

$A^2$ den Rest

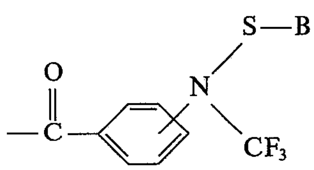

bedeutet
worin

B für Trifluor-methyl, Trichlor-methyl, Difluor-chlor-methyl oder Dichlor-fluor-methyl steht,

$R^1$ und $R^2$ gleich oder verschieden sind und für Wasserstoff, Alkyl mit 1 bis 6 Kohlenstoffatomen, Aryl mit 1 bis 3 linear verknüpften oder kondensierten Phenylkernen stehen oder, für den Fall, das $X^2$ Ethylen bedeutet und $m^2$ und $n^2$ für die Zahl 1 stehen, gemeinsam durch Verknüpfung ebenfalls Ethylen bedeuten,

$m^2$ eine Zahl von 1 bis 5 bedeutet und die Anzahl der Verknüpfungen von

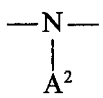

mit $X^2$ angibt und

$n^2$ eine Zahl von 1 bis 20 bedeutet,

genannt.

Besonders bevorzugt werden Fluor- und Chlor-methyl-thioamino-benzamide der Formel

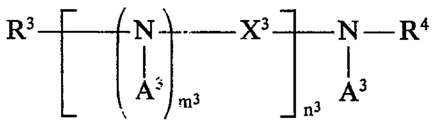

(III)

worin

$X^3$ für Ethylen, Hexylen, Hexylen-2-carbonsäure-methylester, 2-Methylphenylen, Diphenylenmethan, Diphenylenpropan steht,

$A^3$ den Rest

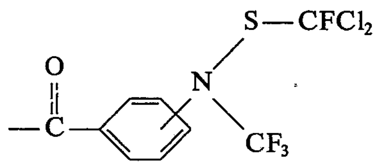

bedeutet,

$R^3$ und $R^4$ gleich oder verschieden sind und Wasserstoff Methyl oder Phenyl bedeuten,

$m^3$ die Zahl 1 oder 2 bedeutet und die Anzahl der Verknüpfungen von

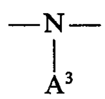

$n^3$ eine Zahl von 1 bis 10 bedeutet.

Im einzelnen seien beispielsweise die folgenden Fluor- und Chlor-methyl-thioamino-benzamide genannt:

Bis-N-(fluordichlormethylthio)-N-(trifluormethyl)-o-amino-benzoesäure-piperazid;
-ethylendiamid;
-(2-methyl-1,5-phenylendiamid);
-(hexylen-2-carbonsäuremethylester-1,6-diamid);
Tris-N-(fluordichlormethylthio)-N-(trifluormethyl)-o-aminobenzoesäure-(4,4',4''-triphenylenmethantriamid);
Hexakis-N-(fluordichlormethylthio)-N-(trifluormethyl)-o-aminobenzoesäure-pentaethylenhexamid;
Bis-N-(fluordichlormethylthio)-N-(trifluormethyl)-m-aminobenzoesäure-piperazid.

Es wurde außerdem ein Verfahren zur Herstellung von Fluor- und Chlor-methyl-thioamino-benzamiden gefunden, das dadurch gekennzeichnet ist, daß man ein Fluor- und Chlor-methyl-thioamino-benzoesäurefluorid der Formel

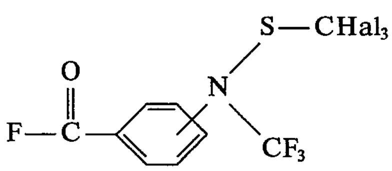

(IV)

worin

Hal die obengenannte Bedeutung hat,

mit einem Amin der Formel

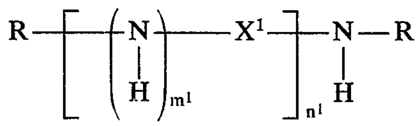

(V)

worin

$X^1$, R, $m^1$ und $n^1$ die obengenannten Bedeutungen haben,

bei erhöhter Temperatur in Gegenwart einer Base umsetzt.

Für das erfindungsgemäße Verfahren können bevorzugt Fluor- und Chlor-methyl-thioamino-benzamide der Formel

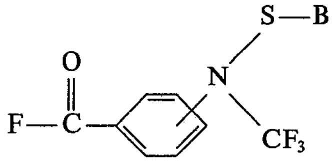

(VI)

worin

B die obengenannte Bedeutung hat,

eingesetzt werden.

Insbesondere bevorzugt werden Verbindungen der folgenden Formel

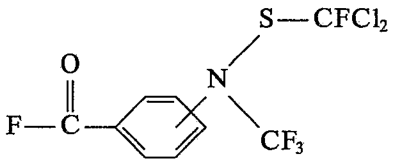

(VII)

eingesetzt.

Im allgemeinen setzt man die meta- und ortho-Isomeren der N-Fluordichlormethylthio-N-trifluormethylaminobenzoesäurefluoride ein.

Die Fluor- und Chlor-methyl-thioamino-benzoesäurefluoride sind an sich bekannt und können beispielsweise durch Umsetzung von N-Trihalogenmethylaminobenzoylfluorid und Halogenmethansulfonsäurechlorid in Gegenwart einer tertiären Base, wie Triethylamin, hergestellt werden (DE-OS 1 293 754).

Beispielsweise seien die folgenden Fluor- und Chlor-methyl-thioamino-benzoesäurefluoride genannt:

N-Trichlormethylthio-N-trifluormethyl-o-amino-benzoesäurefluorid;
N-trifluormethyl-m-amino-benzoesäurefluorid.

Für das erfindungsgemäße Verfahren können bevorzugt Amine der Formel

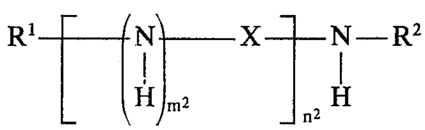

(VIII)

worin

$R^1$, $R^2$, $X^2$, $m^2$ und $n^2$ die obengenannten Bedeutungen haben,

eingesetzt werden.

Insbesondere bevorzugt werden Amine der folgenden Formel

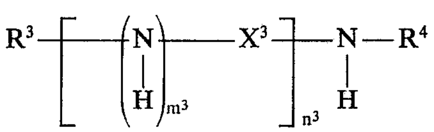

(IX)

worin

$R^3$, $R^4$, $X^3$, $m^3$ und $n^3$ die obengenannten Bedeutungen haben,

eingesetzt.

Die Amine können in an sich bekannter Weise hergestellt werden (Houben-Weyl, Bd. XI/1 [1957]). Beispielsweise kann man sie herstellen durch Umsetzung von Nitroverbindungen mit Reduktionsmitteln, Hydrierung von Nitrilgruppen oder durch sauer katalysierte Polymerisation von Ethylenimin.

Das erfindungsgemäße Verfahren wird im allgemeinen im Temperaturbereich von −20°C bis 100°C, bevorzugt von −10 bis +70°C, durchgeführt.

Im allgemeinen wird das erfindungsgemäße Verfahren bei Normaldruck durchgeführt. Selbstverständlich ist es jedoch auch möglich, die Umsetzung bei einem Überdruck (beispielsweise bis zu 10 bar) oder einem Unterdruck (beispielsweise bis zu 0,1 bar) durchzuführen.

Als Basen für das erfindungsgemäße Verfahren können Säurebindungsmittel eingesetzt werden, die unter den Reaktionsbedingungen den frei werdenden Fluorwasserstoff binden. Beispielsweise seien genannt Alkali- und Erdalkalihydroxide, wie Natriumhydroxid, Kaliumhydroxid, Magnesiumhydroxid und Calciumhydroxid, Alkali- und Erdalkalicarbonate, wie Natriumcarbonat, Magnesiumcarbonat und Calciumcarbonat, Ammoniak, Amine, insbesondere tertiäre Amine der Formel

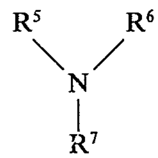

(X)

worin

$R^5$, $R^6$ und $R^7$ gleich oder verschieden sind und für einen niederen geradkettigen oder verzweigten Alkylrest (bis zu etwa 6 Kohlenstoffatomen) oder den Phenylrest stehen.

Bevorzugte Basen sind Triethylamin und N,N-Dimethylbenzylamin.

Das erfindungsgemäße Verfahren kann in einem Lösungs- und/oder Verdünnungsmittel durchgeführt werden. Als Lösungs- und/oder Verdünnungsmittel seien inerte organische Lösungsmittel genannt, die sich unter den Reaktionsbedingungen nicht verändern. Beispielsweise seien Toluol, Dioxan, Aceton, tert.-Butanol oder Isopropanol genannt. Es ist außerdem möglich, das erfindungsgemäße Verfahren in wäßriger Lösung durchzuführen. Die Ausgangsprodukte des erfindungsgemäßen Verfahrens werden im allgemeinen in äquivalenten Mengen eingesetzt. Es ist selbstverständlich möglich die eine oder die andere Reaktionskomponente im Überschuß, beispielsweise bis zu 2 Mol, einzusetzen.

Das Lösungs- und/oder Verdünnungsmittel wird im allgemeinen in einer Menge 20—80 Gew.-%, bezogen auf das Fluor- und Chlormethyl-thioamino-benzamid und das Amin eingesetzt.

Das erfindungsgemäße Verfahren kann beispielsweise wie folgt durchgeführt werden:

Das Fluor- und Chlor-methyl-thioamino-benzamid wird gegebenenfalls mit einem Lösungsmittel verdünnt und anschließend zu einer homogenen Mischung aus einem Molanteil des Amins, mindestens einem Molanteil einer Base und einem Lösungsmittel bei der Reaktionstemperatur langsam unter Rühren zugegeben. Nach beendeter Reaktion wird mit Wasser gewaschen und das erfindungsgemäße Endprodukt abgetrennt.

Die erfindungsgemäße Fluor- und Chlor-methyl-thioamino-benzamide können als Wirkstoffe zur Bekämpfung von Mikroorganismen vorzugsweise in technische Materialien, verwendet werden.

Technische Materialien, die durch die erfindungsgemäßen Wirkstoffe vor einer mikrobiellen Veränderung und Zerstörung geschützt werden sollen, sind beispielsweise Klebstoffe, Leime, Papiere und Kartone, Textilien, Leder, Holz, Anstrichmittel, Putze und Kunststoffartikel, die von Mikroorganismen befallen und/oder zersetzt werden können.

Mikroorganismen, die einen Abbau oder eine Veränderung der technischen Materialien bewirken können, sind beispielsweise Bakterien, Pilze, Hefen, Algen, Schleime und Viren. Vorzugsweise wirken die erfindungsgemäßen Fluor- und Chlor-methyl-thioamino-benzamide gegen Pilze.

Es seien beispielsweise Mikroorganismen der folgenden Gattungen genannt:

Coniophora, wie Coniophora cerebella,
Lentinus, wie Lentinus tigrinus,
Pullularia, wie Pullularia pullulans,
Sclerophoma, wie Sclerophoma pityophila,
Aspergillus, wie Aspergillus niger,
Alternaria, wie Alternaria tenuis,
Chaetomium, wie Chaetomium globosum,
Polyporus, wie Polyporus versicolor,
Penicillium, wie Penicillium glaucum,
Trichoderma, wie Trichoderma viride.

Je nach ihrem Anwendungsgebiet können die erfindungsgemäßen Fluor- und Chlor-methyl-thioamino-benzamide in die üblichen Formulierungen überführt werden, wie Lösungen, Emulsionen, Suspensionen, Pulver, Pasten und Granulate. Diese können in an sich bekannter Weise hergestellt werden, z. B.

durch Vermischen der Wirkstoffe mit einem Streckmittel, mit einem flüssigen Lösungsmittel und/oder festen Trägerstoffen, gegebenenfalls unter Verwendung von oberflächenaktiven Mitteln, wie Emulgatoren und/oder Dispergiermitteln, wobei beispielsweise im Falle der Benutzung von Wasserstreckmitteln, gegebenenfalls organische Lösungsmittel, als Hilfsmittel verwendet werden können.

Flüssige Lösungsmittel der Wirkstoffe können z. B. Alkohole, beispielsweise niedere aliphatische Alkohole, vorzugsweise Ethanol und Isopropanol, und aromatische Alkohole, wie Benzylalkohol, flüssige Kohlenwasserstoffe, wie Benzinfraktionen, chlorierte Kohlenwasserstoffe, wie 1,2-Dichlorethan, Ester wie Diethylenglycoldiacetat oder Ethylacetat, Ketone wie Cyclohexanon oder Aceton, Dimethylformamid oder Dimethylsulfoxid sein.

Feste Trägerstoffe, die bei der Herstellung der fertigen Anwendungsformen des Wirkstoffes zugegeben werden,
können beispielsweise feinteilige Aluminiumoxide, Silikate, Karbonate, Eisenoxide, Gips oder Holzmehl sein.

Oberflächenaktive Mittel können handelsübliche Emulgatoren, wie Aryl- und Alkylsulfonate; ethoxilierte Alkylphenole, Fettsäuren, Fettalkohole oder Alkylamine oder Dispergiermittel, wie Polycarbonsäuren, Polyvinylalkohol, Lignin, Sulfitablaugen oder Methylcellulose sein.

Die Anwendungsform des erfindungsgemäßen mikrobiziden Mittels enthält im allgemeinen 0,1 bis 95 Gew.-%, bevorzugt 0,5 bis 90 Gew.-%, des erfindungsgemäßen Fluor- und Chlor-methyl-thioamino-benzamids als Wirkstoff.

Die für den Schutz technischer Materialien erforderlichen Wirkstoffmengen können in größeren Bereichen variiert werden. Im allgemeinen liegen sie im Bereich von 0,0001 bis 5 Gew.-%, vorzugsweise im Bereich von 0,01 bis 2 Gew.-%, bezogen auf die Gesamtmenge des zu schützenden Materials.

Die erfindungsgemäßen Wirkstoffe können in den Formulierungen in Mischungen mit anderen bekannten anorganischen und organischen Fungiziden, Bakteriziden und/oder Insektiziden vorliegen. Beispielsweise seien die folgenden Wirkstoffe genannt: Benzimidazolylmethyl-carbamat, Tetramethylthiuramdisulfid, p-Chlor-m-kresol, 1-[-Chlorphenyl-bis-(phenyl)-methyl]-imidazol, Parathion, Streptomycin.

A Beispiele zur Herstellung der Fluor- und Chlor-methyl-thioaminobenzamide

Allgemeine Herstellungsvorschrift

0,1 Mol (34 g) N-(Fluordichlormethylthio)-N-(trifluormethyl)-amino-benzoesäurefluorid, wobei in der folgenden Tabelle das ortho-Isomere mit A und das meta-Isomere mit B bezeichnet wird, wird langsam zu einer Lösung aus einem Mol eines Amins und 0,1 Mol Triethylamin in 100 ml t-Butanol zugetropft. Die Temperatur steigt dabei auf ca. 30—35 °C an. Nach beendeter Reaktion wird das Reaktionsgemisch in Eiswasser eingegossen und abfiltriert.

In der folgenden Tabelle sind die Verbindungen zusammengefaßt:

Tabelle 1

| Beispiel Nr. | Säure-fluorid/ Amin | Name der Verbindung | Fp/°C |
|---|---|---|---|
| 1 | A | Bis-N-(fluordichlormethylthio)-N-(trifluormethyl)-O-aminobenzoesäure-(4,4'-diphenylmethandiamid) | 110 |
| 2 | A | Bis-N-(fluordichlormethylthio)-N-(trifluormethyl)-O-aminobenzoesäure-ethylendiamid | 155 |
| 3 | A | Bis-N-(fluordichlormethylthio)-N-(trifluormethyl)-O-aminobenzoesäure-(1,6-hexandiamid) | 161 |
| 4 | A | Bis-N-(fluordichlormethylthio)-N-(trifluormethyl)-O-aminobenzoesäure-(2-methyl-1,5-phenylendiamid) | 175 |
| 5 | A | Bis-N-(fluordichlormethylthio)-N-(trifluormethyl)-O-aminobenzoesäure-(1,4-piperazid) | 180 |
| 6 | A | Bis-N-(fluordichlormethylthio)-N-(trifluormethyl)-O-aminobenzoesäure-(4,4'-diphenylpropandiamid) | 142 |
| 7 | A | Tris-N-(fluordichlormethylthio)-N-(trifluormethyl)-O-aminobenzoesäure-(diethylentriamid) | 122 |
| 8 | A | Bis-N-(fluordichlormethylthio)-N-(trifluormethyl)-O-aminobenzoesäure-(hexylen-2-carbonsäuremethyl-esterdiamid) | 71 |
| 9 | A | Hexakis-N-(fluordichlormethylthio)-N-(trifluormethyl)-O-amino-benzoesäure-(pentaethylenhexamid) | 96 |
| 10 | A | Tris-N-(fluordichlormethylthio)-N-(trifluormethyl)-O-aminobenzoesäure-(4,4',4"-triphenylmethantriamid) | 130 |
| 11 | B | Bis-N-(fluordichlormethylthio)-N-(trifluormethyl)-m-aminobenzoesäure-(4,4'-diphenylmethandiamid) | 97 |
| 12 | B | Bis-N-(fluordichlormethylthio)-N-(trifluormethyl)-m-aminobenzoesäure-(4,4'-diphenylpropandiamid) | 96 |
| 13 | B | Bis-N-(fluordichlormethylthio)-N-(trifluormethyl)-m-aminobenzoesäure-ethylendiamid | 75 |
| 14 | B | Bis-N-(fluordichlormethylthio)-N-(trifluormethyl)-m-aminobenzoesäure-(1,4-piperazid) | Öl |

## B Mikrobizide Wirkung der Fluor- und Chlor-methyl-thioamino-benzamide

### Beispiel 15

In einen Agar, der aus Bierwürze und Pepton hergestellt wurde, wurden erfindungsgemäße Verbindungen jeweils in abgestuften Konzentrationen zwischen 1 und 5000 mg/l je Versuchsprobe eingearbeitet. Nach dem Erstarren des Agars erfolgte die Kontamination der so hergestellten Agarproben mit Reinkulturen verschiedener Testpilze (s. Tabelle 2).

Nach zweiwöchiger Lagerung bei 28 °C und 60 bis 70% relativer Luftfeuchtigkeit wurde ausgewertet. In der Tabelle 2 ist als minimale Hemmkonzentration (MHK) die geringste in einer Agarprobe enthaltende Konzentration einer Substanz angegeben, bei der keinerlei Bewuchs durch die verwendete Art erfolgte.

Tabelle 2

| Testorganismen | MHK-Werte von Fluor- und Chlor-methyl-thioamino-benzamiden in mg/l gemäß Beispiel-Nr. | | | | | | |
|---|---|---|---|---|---|---|---|
| | 1 | 2 | 3 | 4 | 5 | 7 | 14 |
| Coniophora cerebella | 7,5 | 2 | 10 | 1 | 35 | 15 | 5 |
| Lentinus tigrinus | 75 | 20 | 50 | 50 | 100 | 35 | 5 |
| Pullularia pullulans | 150 | 75 | 1500 | 1500 | 750 | 1000 | 5 |
| Sclerophoma pityphila | 150 | 35 | 2000 | 75 | 150 | 200 | 5 |

**Patentansprüche**

1. Fluor- und Chlor-methyl-thioamino-benzamide der Formel

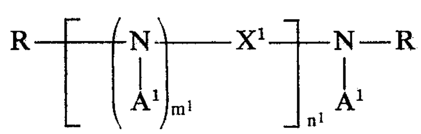

worin

$X^1$ für gegebenenfalls substituiertes Alkylen, Arylen, Alkarylen, wobei die Alkylen- und/oder Arylen- und/oder Alkarylengruppen über Oxo-, Thio-, Carboxo-, Carbonamido- und Sulfoxogruppen verknüpft sein können, steht,

$A^1$ den Rest

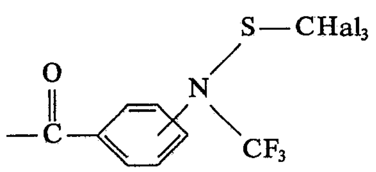

bedeutet,

worin

Hal für Halogen steht,

R in den unterschiedlichen Substitutionen gleich oder verschieden ist und für Wasserstoff, Alkyl oder Aryl steht oder, für den Fall, daß $X^1$ Ethylen bedeutet und $m^1$ und $n^1$ für die Zahl 1 stehen, durch Verknüpfung mit einem zweiten R eine Ethylenbrücke bildet,

$m^1$ eine Zahl von 1 bis 5 bedeutet und die Anzahl der Verknüpfungen von

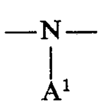

mit $X^1$ angibt und

$n^1$ eine Zahl von 1 bis 100 bedeutet.

2. Fluor- und Chlor-methyl-thioamino-benzamide nach Anspruch 1 der Formel

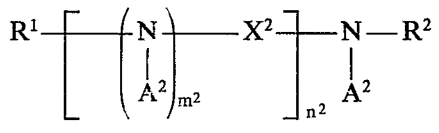

worin

$X^2$ für gegebenenfalls substituiertes geradkettiges oder verzweigtes Alkylen mit 2 bis 6 Kohlenstoffatomen, Arylen mit 1 bis 5 linear verknüpften oder kondensierten Phenylenkernen, Alkarylen, wobei Arylenkerne des Alkarylens durch geradkettiges oder verzweigtes Alkylen mit 1 bis 6 Kohlenstoffatomen verbunden sein können, und wobei Alkylen- und/oder Arylen- und/oder Alkarylengruppen über Oxo-, Thio-, Carboxo-, Carbonamido- und Sulfoxogruppen verknüpft sein können, steht,

$A^2$ den Rest

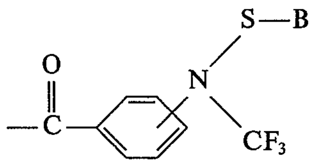

bedeutet,
worin

B für Trifluor-methyl, Trichlor-methyl, Difluorchlor-methyl oder Dichlor-fluor-methyl steht,

$R^1$ und $R^2$ gleich oder verschieden sind und für Wasserstoff, Alkyl mit 1 bis 6 Kohlenstoffatomen, Aryl mit 1 bis 3 linear verknüpften oder kondensierten Phenylkernen stehen oder, für den Fall, das $X^2$ Ethylen bedeutet und $m^2$ und $n^2$ für die Zahl 1 stehen, gemeinsam durch Verknüpfung ebenfalls Ethylen bedeuten,

$m^2$ eine Zahl von 1 bis 5 bedeutet und die Anzahl der Verknüpfungen von

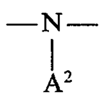

mit $X^2$ angibt und

$n^2$ eine Zahl von 1 bis 20 bedeutet.

3. Fluor- und Chlor-methyl-thioamino-benzamide nach den Ansprüchen 1 und 2 der Formel

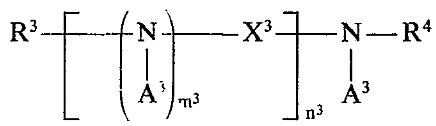

(III)

worin

$X^3$ für Ethylen, Hexylen, Hexylen-2-carbonsäure-methylester, 2-Methylphenylen, Diphenylenmethan, Diphenylenpropan,

$A^3$ den Rest

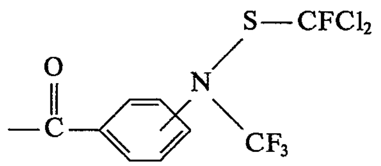

bedeutet,

$R^3$ und $R^4$ gleich oder verschieden sind und Wasserstoff, Methyl oder Phenyl bedeuten,
$m^3$ die Zahl 1 oder 2 bedeutet und die Anzahl der Verknüpfungen von

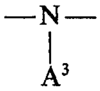

mit $X^3$ angibt und
$n^3$ eine Zahl von 1 bis 10 bedeutet.

4. Verfahren zur Herstellung von Fluor- und Chlor-methyl-thioamino-benzamiden, dadurch gekennzeichnet, daß man ein Fluor- und Chlor-methyl-thioamino-benzoesäurefluorid der Formel

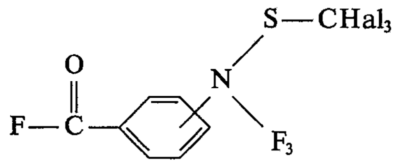

(IV)

worin

Hal für Halogen steht,

mit einem Amin der Formel

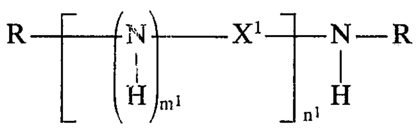

(V)

worin

$X^1$ für gegebenenfalls substituiertes Alkylen, Arylen, Alkarylen, wobei die Alkylen- und/oder Arylen- und/oder Alkarylengruppen über Oxo-, Thio-, Carboxo-, Carbonamido- und Sulfoxogruppen verknüpft sein können, steht,
R in unterschiedlichen Substitutionen gleich oder verschieden ist und für Wasserstoff, Alkyl oder Aryl steht oder, für den Fall, daß $X^1$ Ethylen bedeutet und $m^1$ und $n^1$ für die Zahl 1 stehen, durch Verknüpfung mit einem zweiten R eine Ethylenbrücke bildet,
$m^1$ eine Zahl von 1 bis 5 bedeutet und die Anzahl der Verbindungen von

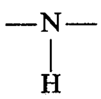

mit $X^1$ angibt und
$n^1$ eine Zahl von 1 bis 100 bedeutet,

bei erhöhter Temperatur in Gegenwart einer Base umsetzt.

5. Verfahren nach Anspruch 4, dadurch gekennzeichnet, daß man die Umsetzung im Temperaturbereich von −20 bis +100°C durchführt.

6. Verfahren nach den Ansprüchen 4 und 5, dadurch gekennzeichnet, daß man als Base Triethylamin oder N,N-Dimethylbenzylamin verwendet.

7. Mikrobizides Mittel, enthaltend Fluor- und Chlor-methyl-thioamino-benzamide der Formel

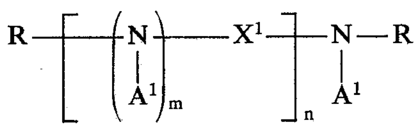

worin

$X^1$ für gegebenenfalls substituiertes Alkylen, Arylen, Alkarylen, wobei die Alkylen- und/oder Arylen- und/oder Alkarylengruppen über Oxo-, Thio-, Carboxo-, Carbonamido- und Sulfoxogruppen verknüpft sein können, steht,

$A^1$ den Rest

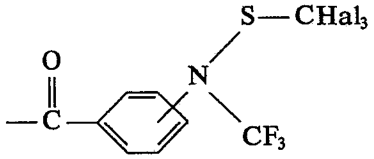

bedeutet,

worin

Hal für Halogen steht,

R in den unterschiedlichen Substitutionen gleich oder verschieden ist und für Wasserstoff, Alkyl oder Aryl steht oder, für den Fall, daß $X^1$ Ethylen bedeutet und $m^1$ und $n^1$ für die Zahl 1 stehen, durch Verknüpfung mit einem zweiten R eine Ethylenbrücke bildet,

$m^1$ eine Zahl von .1 bis 5 bedeutet und die Anzahl der Verbindungen von

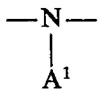

mit $X^1$ angibt und

$n^1$ eine Zahl von 1 bis 100 bedeutet.

8. Mikrobizides Mittel nach Anspruch 7, enthaltend 0,1 bis 95 Gew.-%des Fluor- und Chlor-methyl-thioamino-benzamids.

9. Verwendung von Fluor- und Chlor-methyl-thioamino-benzamiden der Formel

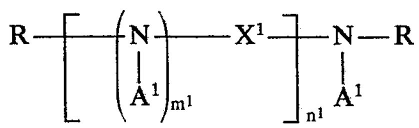

worin

$X^1$ für gegebenenfalls substituiertes Alkylen, Arylen, Alkarylen, wobei die Alkylen- und/oder Arylen- und/oder Alkarylengruppen über Oxo-, Thio-, Carboxo-, Carbonamido- und Sulfoxogruppen verknüpft sein können, steht,

$A^1$ den Rest

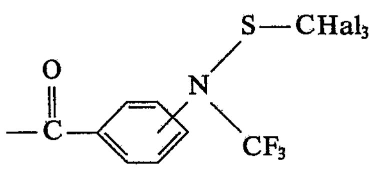

bedeutet,

worin

Hal für Halogen steht,

R in den unterschiedlichen Substitutionen gleich oder verschieden ist und für Wasserstoff, Alkyl oder Aryl steht oder, für den Fall, daß $X^1$ Ethylen bedeutet und $m^1$ und $n^1$ für die Zahl 1 stehen, durch Verknüpfung mit einem zweiten R eine Ethylenbrücke bildet,

$m^1$ eine Zahl von 1 bis 5 bedeutet und die Anzahl der Verbindungen von

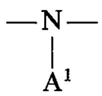

mit $X^1$ angibt und
$n^1$ eine Zahl von 1 bis 100 bedeutet.

zum Schutz technischer Materialien.

**Claims**

1. Fluoro- and chloro-methyl-thioamino-benzamides of the formula

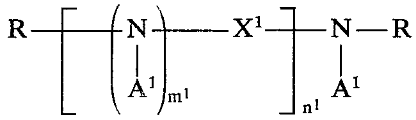

wherein

$X^1$ represents optionally substituted alkylene, arylene or alkarylene, it being possible for the alkylene and/or arylene and/or alkarylene groups to be linked via oxo, thio, carboxo, carboxamido or sulphoxo groups,
$A^1$ denotes the radical

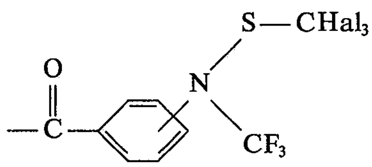

wherein

Hal represents halogen,

the various substituents R can be identical or different and represent hydrogen, alkyl or aryl or a substituent R, in the case where $X^1$ denotes ethylene and $m^1$ and $n^1$ represent the number 1, forms an ethylene bridge by being linked to the second R,
$m^1$ denotes an integer from 1 to 5 and indicates the number of linkages of

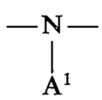

with $X^1$, and
$n^1$ denotes a number from 1 to 100.

2. Fluoro- and chloro-methyl-thioamino-benzamides according to Claim 1, of the formula

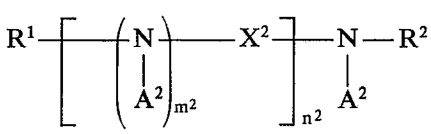

wherein

$X^2$ represents optionally substituted straight-chain or branched alkylene with 2 to 6 carbon atoms, arylene which has 1 to 5 phenylene nuclei linked in a linear manner or fused together, or alkarylene, it being possible for the arylene nuclei of the alkarylene to be linked by straight-chain or branched alkylene with 1 to 6 carbon atoms, and it being possible for the alkylene and/or arylene and/or alkarylene groups to be linked via oxo, thio, carboxo, carboxamido or sulphoxo groups,
$A^2$ denotes the radical

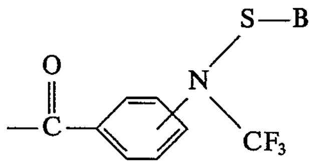

wherein

B represents trifluoro-methyl, trichloro-methyl, difluoro-chloro-methyl or dichloro-fluoro-methyl,

$R^1$ and $R^2$ are identical or different and represents hydrogen, alkyl with 1 to 6 carbon atoms or aryl with 1 to 3 phenyl nuclei linked in a linear manner or fused together, or $R^1$ and $R^2$, in the case where $X^2$ denotes ethylene and $m^2$ and $n^2$ represent the number 1, together also denote ethylene by being linked together,

$m^2$ denotes a number from 1 to 5 and indicates the number of linkages of

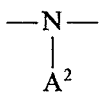

with $X^2$ and

$n^2$ denotes a number from 1 to 20.

3. Fluoro- and chloro-methyl-thioamino-benzamides according to Claims 1 and 2, of the formula

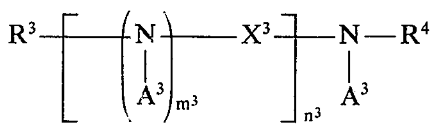

(III)

wherein

$X^3$ represents ethylene, hexylene-2-carboxylic acid methyl ester, 2-methylphenylene di-phenylenemethane or diphenylenepropane,

$A^3$ denotes the radical

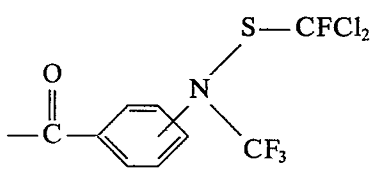

$R^3$ and $R^4$ are identical or different and denote hydrogen, methyl or phenyl,

$m^3$ denotes the number 1 or 2 and indicates the number of linkages of

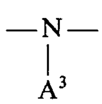

with $X^3$ and

$n^3$ denotes a number from 1 to 10.

4. Process for the preparation of fluoro- and chloro-methyl-thioamino-benzamides, characterised in that a fluoro- or chloro-methyl-thioamino-benzoic acid fluoride of the formula

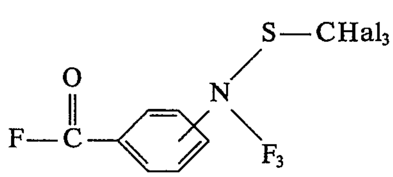

(IV)

wherein

Hal represents halogen,

is reacted with an amine of the formula

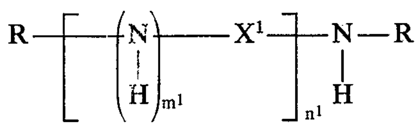

(V)

wherein

$X^1$ represents optionally substituted alkylene, arylene or alkarylene, it being possible for the alkylene and/or arylene and/or alkarylene groups to be linked via oxo, thio, carboxo, carboxamido or sulphoxo groups,

the various substituents R can be identical or different and represent hydrogen, alkyl or aryl or a substituent R, in the case where $X^1$ denotes ethylene and $m^1$ and $n^1$ represent the number 1, forms an ethylene bridge by being linked to a second R,

$m^1$ denotes an integer from 1 to 5 and indicates the number of linkages of

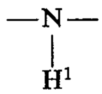

with $X^1$, and

$n^1$ denotes a number from 1 to 100,

at elevated temperature in the presence of a base.

5. Process according to Claim 4, characterised in that the reaction is carried out in the temperature range from −20 to +100°C.

6. Process according to Claims 4 and 5, characterised in that triethylamine or N,N-dimethylbenzylamine is used as the base.

7. Microbicidal agent containing a fluoro- or chloro-methyl-thioamino-benzamide of the formula

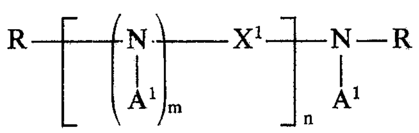

wherein

$X^1$ represents optionally substituted alkylene, arylene or alkarylene, it being possible for the alkylene and/or arylene and/or alkarylene groups to be linked via oxo, thio, carboxo, carboxamido or sulphoxo groups,

$A^1$ denotes the radical

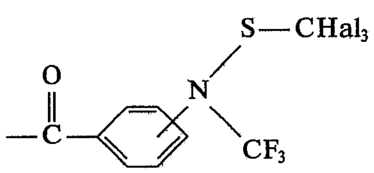

wherein

Hal represents halogen,

the various substituents R can be identical or different and represent hydrogen, alkyl or aryl or a substituent R, in the case where $X^1$ denotes ethylene and $m^1$ and $n^1$ represent the number 1, forms an ethylene bridge by being linked to a second R,

$m^1$ denotes an integer from 1 to 5 and indicates the number of linkages of

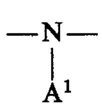

with $X^1$, and

$n^1$ denotes a number from 1 to 100.

8. Microbicidal agent according to Claim 7, containing 0.1 to 95 % by weight of the fluoro- or chloro-methyl-thioamino-benzamide.

9. Use of fluoro- and chloro-methyl-thioamino-benzamides of the formula

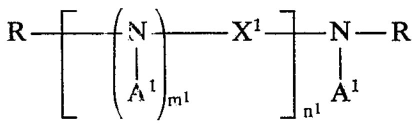

wherein

$X^1$ represents optionally substituted alkylene, arylene or alkarylene, it being possible for the alkylene and/or arylene and/or alkarylene groups to be linked via oxo, thio, carboxo, carboxamido or sulphoxo groups,

$A^1$ denotes the radical

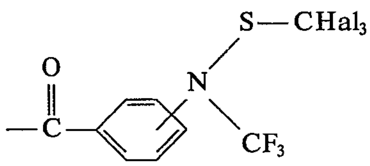

wherein

Hal represents halogen,

the various substituents R can be identical or different and represent hydrogen, alkyl or aryl or a substituent R, in the case where $X^1$ denotes ethylene and $m^1$ and $n^1$ represent the number 1, forms an ethylene bridge by being linked to a second R,

$m^1$ denotes an integer from 1 to 5 and indicates the number of linkages of

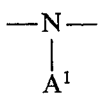

with $X^1$, and

$n^1$ denotes a number from 1 to 100,

for protecting industrial materials.

**Revendications**

1. Fluoro- et chloro-méthyl-thioamino-benzamides de formule

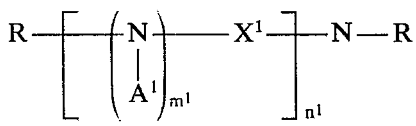

(I)

dans laquelle

$X^1$ représente un groupe alkylène, arylène, alkarylène, éventuellement substitué, les groupes alkylènes et/ou arylènes et/ou alkarylènes pouvant être reliés par l'intermédiaire de groupes oxo, thio, carboxo, carbonamido et sulfoxo,

$A^1$ représente le reste

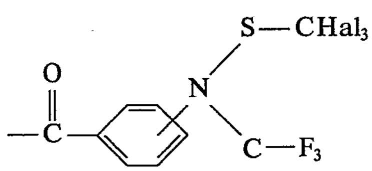

dans laquelle

Hal représente un halogène,

R est le même ou différent dans les diverses substitutions et représente l'hydrogène ou un groupe alkyle ou aryle ou bien, dans le cas où $X_1$ représente un groupe éthylène et $m^1$ et $n^1$ sont égaux à 1, le reste R forme un pont éthylène par jonction avec un second reste R,

$m^1$ représente un entier de 1 à 5 et indique le nombre de jonctions de

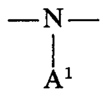

avec $X^1$ et

$n^1$ représente un nombre de 1 à 100.

2. Fluoro- et chloro-méthyl-thioamino-benzamides selon la revendication 1 de formule

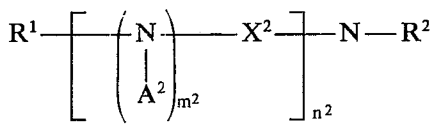

(II)

dans laquelle

$X^2$ représente un reste alkylène à chaîne droite ou ramifiée en $C_2$—$C_6$, éventuellement substitué, arylène ayant 1 à 5 noyaux phénylènes reliés linéairement ou condensés, alkarylène, les noyaux arylènes du reste alkarylène pouvant être reliés par un reste alkylène à chaîne droite ou ramifiée en $C_1$—$C_6$, et les groupes alkylènes et/ou arylènes et/ou alkarylènes pouvant être reliés par l'intermédiaire de groupes oxo, thio, carboxo, carbonamido et sulfoxo,

$A^2$ représente le reste

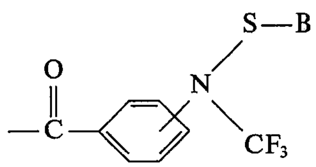

dans laquelle

B représente un reste trifluorométhyle, trichlorométhyle, difluorochlorméthyle ou dichlorofluorométhyle,

$R^1$ et $R^2$ sont identiques ou différents et représentent l'hydrogène ou un reste alkyle en $C_1$—$C_6$, aryle ayant 1 à 3 noyaux phényles reliés linéairement ou condensés ou bien, dans le cas où $X^2$ représente le reste éthylène et $m^2$ et $n^2$ sont égaux à 1, $R^1$ et $R^2$ reliés ensemble forment également un reste éthylène,

$m^2$ est un nombre de 1 à 5 et indique le nombre de jonctions de

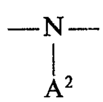

avec $X^2$ et

$n^2$ est un nombre de 1 à 20.

3. Fluoro- et chloro-méthyl-thioamino-benzamides selon les revendications 1 et 2 de formule

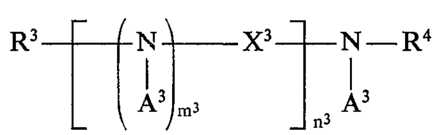

(III)

dans laquelle

$X^3$ représente un reste éthylène, hexylène, hexylène-2-carboxylate de méthyle, 2-méthyl-phénylène, diphénylèneméthane, diphénylpropane,

$A^3$ représente le reste

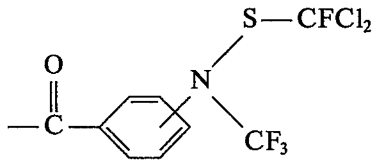

$R^3$ et $R^4$ sont identiques ou différents et représentent l'hydrogène ou un reste méthyle ou phényle,

$m^3$ représente le nombre 1 ou 2 et indique le nombre de jonctions de

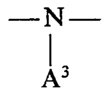

avec $X^3$ et

$n^3$ représente un nombre de 1 à 10.

4. Procédé pour la fabrication de fluoro- et chloro-méthyl-thioamino-benzamides, caractérisé en ce que l'on fait réagir un fluorure d'acide fluoro- et chloro-méthyl-thioamino-benzoïque de formule

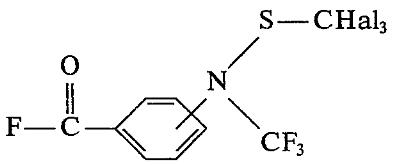

(IV)

dans laquelle

Hal représente un halogène,

avec une amine de formule

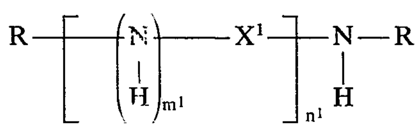

(V)

dans laquelle

$X^1$ représente un groupe alkylène, arylène, alkarylène, éventuellement substitué, les groupes alkylènes et/ou arylènes et/ou alkarylènes pouvant être reliés par l'intermédiaire de groupes oxo, thio, carboxo, carbonamido et sulfoxo,

R est le même ou différent dans les diverses substitutions et représente l'hydrogène ou un groupe alkyle ou aryle, ou bien, dans le cas où $X_1$ représente un groupe éthylène et $m^1$ et $n^1$ sont égaux à 1, le reste R forme un pont éthylène par jonction avec un second reste R,

$m^1$ représente un entier de 1 à 5 et indique le nombre de combinaisons de

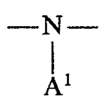

avec $X^1$ et

$n^1$ représente un nombre de 1 à 100.

5. Procédé selon la revendication 4, caractérisé en ce que l'on effectue la réaction dans l'intervalle de températures de −20 à +100°C.

6. Procédé selon la revendication 4 ou 5, caractérisé en ce que l'on utilise comme base la triéthylamine ou la N,N-diméthylbenzylamine.

7. Agent microbicide, contenant des fluoro- et chloro-méthyl-thioamino-benzamides de formule

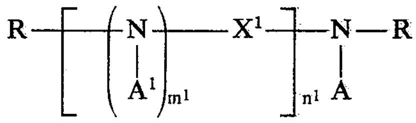

(I)

dans laquelle

$X^1$ représente un groupe alkylène, arylène, alkarylène, éventuellement substitué, les groupes alkylènes et/ou arylènes et/ou alkarylènes pouvant être reliés par l'intermédiaire de groupes oxo, thio, carboxo, carbonamido et sulfoxo,

$A^1$ représente le reste

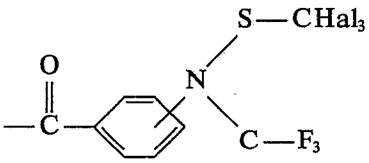

dans laquelle

Hal représente un halogène,

R est le même ou différent dans les diverses substitutions et représente l'hydrogène ou un groupe alkyle ou aryle ou bien, dans le cas où $X_1$ représente un groupe éthylène et $m^1$ et $n^1$ sont égaux à 1, le reste R forme un pont éthylène par jonction avec un second reste R,

$m^1$ représente un entier de 1 à 5 et indique le nombre de combinaisons de

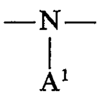

avec $X^1$ et

$n^1$ représente un nombre de 1 à 100.

8. Agent microbicide selon la revendication 7 contenant 0,1 à 95% en poids du fluoro- et chloro-méthyl-thioamino-benzamide.

9. Utilisation de fluoro- et chloro-méthyl-thioamino-benzamides de formule

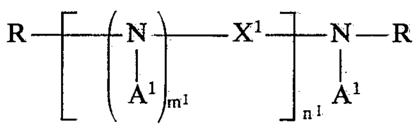

(I)

dans laquelle

$X^1$ représente un groupe alkylène, arylène, alkarylène, éventuellement substitué, les groupes alkylènes et/ou arylènes et/ou alkarylènes pouvant être reliés par l'intermédiaire de groupes oxo, thio, carboxo, carbonamido et sulfoxo,

$A^1$ représente le reste

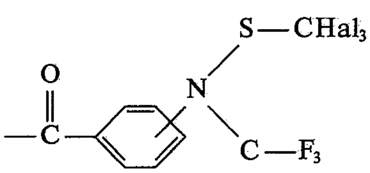

dans laquelle

Hal représente un halogène,

R est le même ou différent dans les diverses substitutions et représente l'hydrogène ou un groupe alkyle ou aryle ou bien, dans le cas où $X_1$ représente un groupe éthylène et $m^1$ et $n^1$ sont égaux à 1, le reste R forme un pont éthylène par jonction avec un second reste R,

$m^1$ représente un entier de 1 à 5 et indique le nombre de combinaisons de

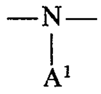

avec $X^1$ et

$n^1$ représente un nombre de 1 à 100,

pour la protection de matériaux techniques.